Europäisches Patentamt

European Patent Office

Office européen des brevets

(11) Veröffentlichungsnummer: **0 113 835**
**A1**

(12) # EUROPÄISCHE PATENTANMELDUNG

(21) Anmeldenummer: 83111687.6

(22) Anmeldetag: 23.11.83

(51) Int. Cl.³: **C 07 H 9/04,** C 07 D 307/62

(30) Priorität: 11.12.82 DE 3245920

(43) Veröffentlichungstag der Anmeldung: **25.07.84**
**Patentblatt 84/30**

(84) Benannte Vertragsstaaten: **CH DE GB LI**

(71) Anmelder: **Merck Patent Gesellschaft mit beschränkter Haftung, Frankfurter Strasse 250, D-6100 Darmstadt (DE)**

(72) Erfinder: **Wintermeyer, Willi, Dr., Gartenstrasse 4, D-6104 Seeheim-Jugenheim (DE)**
Erfinder: **Reiff, Friedrich, Dr., Im Rassdorf 6, D-6104 Seeheim 1 (DE)**
Erfinder: **Butzke, Jürgen, Waldstrasse 48, D-6110 Dieburg (DE)**
Erfinder: **Wittmann, Rolf Karl Otto, Dr., Verstorben (DE)**
Erfinder: **Wolf, Rudolf, Dr., Moserstrasse 6, D-6100 Darmstadt (DE)**

(54) **Verfahren zur Aufreinigung von Diacetonsorbose.**

(57) Ein Verfahren zur Aufreinigung von Diacetonsorbose in ihrer bei der Acetonierung von Sorbose nach Extraktion mit einem organischen Lösungsmittel anfallenden Lösung, wobei man diese Lösung zunächst mit wässeriger Alaklilauge wäscht und nach der Abtrennung der wässerigen alkalischen Phase durch Verteilung zwischen Wasser oder schwach alkalischen wässerigen Lösungen und einem organischen Lösungsmittel eine reine Diacetonsorboselösung erhält, die für die Herstellung von Ascorbinsäure verwendet werden kann.

EP 0 113 835 A1

0113835

Merck Patent Gesellschaft
mit beschränkter Haftung


Verfahren zur Aufreinigung von Diacetonsorbose

Die Erfindung betrifft ein Verfahren zur Aufreinigung von Diacetonsorbose, einem Zwischenprodukt der Vitamin C-Synthese.

Bei der Herstellung von Diaceton-2-ketogulonsäure ist die Reinheit der eingesetzten Diacetonsorbose entscheidend für die Ausbeute und vor allem für den Verbrauch von Oxidationsmitteln wie etwa von Bleichlauge oder von elektrischem Strom.

Bei der Acetonierung von Sorbose nach den geläufigen Verfahren mit Aceton und Schwefelsäure oder anderen Katalysatoren werden stets Mischungen von Diacetonsorbosen, Monoacetonsorbosen sowie von höher acetonierten Sorbosen und anderen Nebenprodukten erhalten.

Beim heutigen Standardverfahren wird nach der Acetonierung die Schwefelsäure in der Reaktionslösung durch wässerige Alkalilauge neutralisiert, das überschüssige Aceton abdestilliert und die Diacetonsorbose mit einem organischen Lösungsmittel extrahiert. Die aus dem

GSPHA32 F-fi

organischen Lösungsmittel gewonnene Diacetonsorbose enthält neben anderen Verunreinigungen auch Monoacetonsorbosen sowie höher acetonierte Nebenprodukte. Der Einsatz so erhaltener roher Diacetonsorbose erfordert in der Oxidationsstufe sehr hohe Oxidationsmittelmengen, weil die Verunreinigungen dabei weitgehend durchoxidiert und abgebaut werden.

Nach dem Stand der Technik (siehe z. B. Helv.Chim.Acta 17, 311, 1934, DBP 1618885, DE-OS 2003067) wird die Diacetonsorbose aus dem Reaktionsgemisch gewöhnlich mit einem organischen Lösungsmittel wie Diethylether, Benzol, Toluol, Chloroform oder 1,2-Dichlorethan extrahiert und mit Wasser, Pottasche-Lösung oder stark verdünnter Natronlauge gewaschen. Da alle Produkte sowohl in organischen Lösungsmitteln als auch in Wasser löslich sind, verlaufen solche Waschprozesse nicht besonders selektiv, was zur Folge hat, daß damit keine wirksame Aufreinigung verbunden ist.

Aufgabe der vorliegenden Erfindung war es, reine Diacetonsorbose mit hoher Ausbeute bereitzustellen, um optimale Voraussetzungen für die Oxidationsstufe der Ascorbinsäuresynthese zu schaffen.

Es wurde nun gefunden, daß durch Waschen der in dem organischen Lösungsmittel gelösten, rohen Diacetonsorbose mit Alkalilauge, gefolgt von einer Verteilung der verunreinigten Diacetonsorbose zwischen Wasser oder schwach alkalischen wässerigen Lösungen und einem organischen Lösungsmittel, die bei der Acetonierung gebildeten Nebenprodukte nahezu quantitativ abgetrennt und in den Acetonierungsprozeß zurückgeführt werden können, wodurch man in hoher Ausbeute eine sehr reine Diacetonsorbose erhält.

Gegenstand der Erfindung ist demnach das Verfahren zur Aufreinigung von Diacetonsorbose in ihren bei der Acetonierung von Sorbose nach Extraktion mit einem organischen Lösungsmittel anfallenden Lösungen, bei dem man die organische Phase zunächst mit wässeriger Alkalilauge wäscht und nach der Abtrennung der wässerigen alkalischen Phase durch Verteilung zwischen Wasser oder schwach alkalischen wässerigen Lösungen und einem organischem Lösungsmittel reine Diacetonsorbose erhält.

Ausgangspunkt des erfindungsgemäßen Verfahrens ist die rohe Diacetonsorboselösung, die durch Acetonierung von Sorbose mit Aceton in Gegenwart von Schwefelsäure oder einem anderen Katalysator, Neutralisation der erhaltenen Reaktionslösung mit Alkalibase, Abdestillieren des Acetons und nachfolgender Extraktion mit einem organischen Lösungsmittel erhalten wird. Als organische Lösungsmittel zur Extraktion der Diacetonsorbose können alle gegen Alkalien widerstandfähigen, mit Wasser nicht mischbaren Lösungsmittel, beispielsweise aromatische Kohlenwasserstoffe wie Xylol, Toluol, Benzol, Chlorbenzol, Alkohole wie Butanole, z. B. n-Butanol, Isobutanol, sek.-Butanol oder Amylalkohole wie Pentanol-1, Pentanol-2, Pentanol-3, Isobutylcarbinol, Methylisopropylcarbinol, sek.-Butylcarbinol, Ether wie Diethylether, Dipropylether, Dibutylether, Methyl-tert.-butylether, chlorierte Kohlenwasserstoffe wie Methylendichlorid, Chloroform, 1,2-Dichlorethan, Tetrachlorkohlenstoff oder Trichlorethan verwendet werden. Aromatische Kohlenwasserstoffe werden im allgemeinen bevorzugt.

Diese in einem organischen Lösungsmittel gelöste rohe Diacetonsorbose (Konzentration der Lösungen üblicherweise etwa 10 - 60 Gew.% Diacetonsorbose) wird erfindungsgemäß mit Alkalilauge gewaschen. Dabei werden die Monoacetonsorbosen und gefärbte Verunreinigungen weitgehend abgetrennt. Die Diacetonsorbose bleibt jedoch überraschenderweise trotz ihrer guten Wasserlöslichkeit zum weit überwiegenden Teil in der organischen Phase. Bei der Waschung mit stark verdünnter, 0,05 molarer Natronlauge, wie sie etwa aus der DE-OS 2 003 067 bekannt ist, muß, bedingt durch die hohe Löslichkeit von Diacetonsorbose in 0,05 molarer Natronlauge, mit erheblichen Verlusten an Diacetonsorbose gerechnet werden.

Die Waschung mit Alkalilaugen wird vorzugsweise bei erhöhter Temperatur von 20 bis 80 Grad Celsius, vorzugsweise bei 40 bis 60 Grad Celsius durchgeführt.

Als wässerige Alkalilaugen können etwa 2 bis 50prozentige, vorzugsweise 10 bis 40prozentige, insbesondere 15 bis 30prozentige Natrium- oder Kaliumhydroxid-Lösungen verwendet werden, die in einer Menge von etwa 2 bis 20 l, vorzugsweise 5 bis 10 l, pro 100 l der organischen Diacetonsorboselösung eingesetzt werden. Die Waschung mit wässeriger Alkalilauge kann ein- oder mehrstufig, vorzugsweise jedoch zwei- oder dreistufig erfolgen.

In einer bevorzugten Ausführungsform der Erfindung wird die mit Alkalilauge gewaschene Diacetonsorboselösung, die noch andere Acetonierungs-Nebenprodukte enthält, anschließend mit Wasser oder schwach alkalischen wässerigen Lösungen extrahiert. Die Extraktion wird in der Kälte oder unter nur geringer Erwärmung in einem von etwa 0 bis 50 Grad Celsius reichenden Temperaturbereich, vorzugsweise bei 5 bis 30 Grad Celsius durchgeführt. Die Wassermenge kann etwa 100 bis etwa 300 l, vorzugsweise 150 bis 250 l pro 100 l organischer Diacetonsorboselösung betragen. Dabei wird überraschenderweise praktisch nur die Diacetonsorbose in die wässerige Phase überführt, während noch vorhandene Acetonierungs-Nebenprodukte praktisch quantitativ in der organischen Phase

- 5 -

verbleiben. Die erhaltene reine wässerige Diacetonsorboselösung kann direkt der Oxidationsstufe der Ascorbinsäuresynthese zugeführt werden. Die Acetonierungs-Nebenprodukte setzen sich aus Acetonkondensationsprodukten
und höher kondensierten Sorbosederivaten, die 3 bis 5
Aceton-Einheiten und 1 bis 2 Sorbose-Einheiten enthalten
können, zusammen. Die genaue Zusammensetzung der Acetonierungs-Nebenprodukte ist nicht kritisch, da sie
ohnehin in den Acetonierungsprozeß rückgeführt werden
und letztlich weitestgehend in Diacetonsorbose übergeführt werden.

In einer weiteren bevorzugten Ausführungsform der Erfindung wird die mit Alkalilauge gewaschene Diacetonsorboselösung, die vor allem noch Acetonierungs-Nebenprodukte
enthält, zunächst vom organischen Lösungsmittel befreit,
der Rückstand in Wasser oder schwach alkalischen
wässerigen Lösungen gelöst und die wässerige Lösung
anschließend mit einem organischen Lösungsmittel
gewaschen.

Als organische Lösungsmittel können im Prinzip alle mit
Wasser nicht mischbaren Lösungsmittel verwendet werden.
Aus Zweckmäßigkeitsgründen werden jedoch solche Lösungsmittel bevorzugt, die leicht rückgewinnbar und wenig
toxisch sind, beispielsweise Alkohole wie Butanole, z.B.
n-Butanol, Isobutanol, sek.-Butanol oder Amylalkohole
wie Pentanol-1, Pentanol-2, Pentanol-3, Isobutylcarbinol,
Methylisopropylcarbinol, sek.-Butylcarbinol, Ether wie
Diethylether, Dipropylether, Dibutylether oder Methyl-
tert.-butylether. Besonders bevorzugt sind aliphatische
Kohlenwasserstoffe, wie z. B. Pentane, Hexane, Heptane,
Oktane, Nonane und Decane, cycloaliphatische Kohlenwasserstoffe, wie z. B. Cyclopentan, Cyclohexan und Methylcyclohexan, aromatische Kohlenwasserstoffe, wie z. B.

GSPHA32 F-fi

Benzol, Toluol, Xylol und Ethylbenzol, chlorierte Kohlenwasserstoffe wie Methylendichlorid, Chloroform, Tetrachlorkohlenstoff, Dichlorethan und Trichlorethan sowie
Gemische der angegebenen Verbindungen, die in einer Menge
von etwa 0,2 bis 5 l vorzugsweise 0,4 bis 2 l pro 100 l
der wässerigen rohen Diacetonsorboselösung bei einer
Wasch-Temperatur von 10 bis 50 Grad Celsius, vorzugsweise
bei 20 bis 40 Grad Celsius, eingesetzt werden. Dabei
werden die Acetonierungs-Nebenprodukte praktisch quantitativ in die organische Phase überführt. Die erhaltene
reine wässerige Diacetonsorboselösung kann direkt oder
nach kurzem Andestillieren zur Entfernung von Restlösungsmitteln der Oxidationsstufe der Ascorbinsäuresynthese
zugeführt werden.

In einer bevorzugten Ausführungsform der Erfindung werden
sowohl die wässerigen, alkalischen Phasen aus der Waschung
der organischen Diacetonsorbose-Phase mit Alkalilauge als
auch die die anderen Acetonierungs-Nebenprodukte enthaltenden Rückstände in den Acetonierungsprozeß rückgeführt.

Hierzu wird die wässerige alkalische Phase, welche bei
der Extraktion der organischen Phase mit Alkalilauge erhalten wird und neben Alkalihydroxyd etwas Diacetonsorbose, Monoacetonsorbose und andere Nebenprodukte enthält, in den Prozeß, vorzugsweise an der Stelle der Neutralisation der Reaktionslösung der Sorboseacetonierung,
zurückgeführt.

Der Rückstand der organischen Phase aus der Verteilung
der verunreinigten Diacetonsorbose zwischen Wasser oder
schwach alkalischen wässerigen Lösungen, der vorwiegend
aus höher acetonierten Derivaten der Sorbose sowie aus Acetonkondensationsprodukten besteht und zum größten Teil noch Wertprodukt darstellt, wird ebenfalls in den Acetonierungsprozeß, vorzugsweise in acetonischer Lösung zusammen mit der Monoacetonsorbose, zurückgeführt.

Auf diese Weise bleiben alle Wertprodukte einschließlich dem Alkalihydroxyd im Kreislauf und werden optimal ausgenutzt.

Die in jedem Fall erhaltene reine wässerige Diacetonsorbose wird nun direkt mit Bleichlauge, elektrochemisch oder mit katalytisch aktiviertem Sauerstoff zu Diaceton-2-keto-gulonsäure oxidiert. Durch den Einsatz der erfindungsgemäß gereinigten Diacetonsorbose werden bei der Oxidation gegenüber dem bisherigen Verfahren erhebliche Mengen Oxidationsmittel eingespart.

Damit wird durch die vorliegende Erfindung ein vorteilhaftes neues Verfahren zur Aufreinigung von Diacetonsorbose geliefert, wodurch innerhalb der Vitamin C Synthese erhebliche Verbesserungen erzielt werden.

Beispiel 1

100 kg einer rohen xylolischen Diacetonsorbose-Lösung, die durch die Acetonierung von Sorbose mit Aceton/ Schwefelsäure, Neutralisation der dabei anfallenden Reaktionslösung mit Natronlauge und Extraktion der schwach basischen Reaktionsmischung mit Xylol gewonnen wurde, und die neben etwa 33 Gew.% Diacetonsorbose noch Monoacetonsorbosen, höher acetonierte Sorbosederivate, andere Nebenprodukte sowie stark gefärbte Verunreinigungen enthält, wird dreimal mit je 3,2 kg wässeriger 10 Gew.%iger Natronlauge bei 25 °C gewaschen. Die je-

weils abgetrennten, anfangs stark gefärbten wässerigen Phasen werden vereinigt und zur Neutralisation der Schwefelsäure bei der Aufarbeitung der Acetonierungsansätze verwendet.

Die xylolische Diacetonsorbose-Lösung wird bei Raumtemperatur mit 200 kg Wasser in einer Gegenstrom-Rührzellenextraktionskolonne extrahiert. Die abgetrennte wässerige Lösung enthält 14 Gew.% Diacetonsorbose.

Diese Diacetonsorbose-Lösung, die gegebenenfalls zur Abtrennung von Restxylol andestilliert wird, ist frei von Verunreinigungen und führt bei der nachfolgenden Oxidation zu einer sehr reinen Diaceton-2-ketogulonsäure. Die in der Xylol-Lösung verbliebenen Bestandteile werden nach Abdestillieren des Lösungsmittels in Aceton gelöst und der Acetonierung der Sorbose zugeführt.

Beispiel 2

600 kg einer analog Beispiel 1 gewonnenen rohen 42.5 %igen Diacetonsorbose-Lösung in Toluol wird in einer Rührzellen-Extraktionskolonne dreimal mit je 11 kg 20 Gew.%iger Kalilauge bei 50 °C im Gegenstrom gewaschen. Die wässerigen alkalischen Phasen werden vereinigt und in den Acetonierungsprozeß rückgeführt.

Nach Abkühlung auf Raumtemperatur wird das xylolische Raffinat mit 900 1 Wasser in einer Rührzellen-Extraktionskolonne bei 10 °C extrahiert. Die wässerige Lösung, enthaltend 22 Gew.% Diacetonsorbose, wird anschließend der Oxidation zugeführt.

Beispiel 3

600 kg einer analog Beispiel 1 gewonnenen 38 %igen Diacetonsorbose-Lösung in Toluol wird dreimal mit je 8 kg 30 %iger Natronlauge bei 60 °C gewaschen. Nach Abtrennung der wässerigen alkalischen Phasen wird das organische Lösungsmittel entfernt, der Rückstand in 1200 kg Wasser gelöst und mit 10 l Cyclohexan gewaschen. Die wässerige Phase enthält 16 % Diacetonsorbose und wird nach dem Abdestillieren von Cyclohexanspuren der Oxidation zugeführt.

Der Rückstand der Cyclohexan-Phase nach Abdestillieren des Lösungsmittels sowie die wässerigen alkalischen Phasen werden in den Acetonierungsprozeß rückgeführt.

Merck Patent Gesellschaft
mit beschränkter Haftung


P a t e n t a n s p r ü c h e


1.  Verfahren zur Aufreinigung von Diacetonsorbose in
    ihrer bei der Acetonierung von Sorbose nach Extraktion mit einem organischen Lösungsmittel anfallenden Lösung, dadurch gekennzeichnet, daß man die
    organische Phase zunächst mit wässeriger Alkalilauge
    wäscht und nach der Abtrennung der wässerigen alkalischen Phase durch Verteilung zwischen Wasser oder
    schwach alkalischen wässerigen Lösungen und einem
    organischen Lösungsmittel reine Diacetonsorboselösungen erhält.


2.  Verfahren nach Anspruch 1, dadurch gekennzeichnet,
    daß man nach Abtrennung der wässerigen alkalischen Phase die organische Phase mit Wasser oder
    schwach alkalischen wässerigen Lösungen extrahiert,
    wobei eine wässerige Lösung von reiner Diacetonsorbose erhalten wird.


3.  Verfahren nach Anspruch 1, dadurch gekennzeichnet,
    daß man nach Abtrennung der wässerigen alkalischen Phase das organische Lösungsmittel abdestilliert, den Rückstand in Wasser oder schwach alkalischen wässerigen Lösungen löst und die so erhaltene wässerige Lösung mit einem organischen Lösungsmittel wäscht, wobei eine wässerige Lösung von reiner Diacetonsorbose erhalten wird.

4. Verfahren nach einem der Ansprüche 1 - 3, dadurch gekennzeichnet, daß man die organische Phase mit 2 bis 50prozentiger Natronlauge oder Kalilauge wäscht.

5. Verfahren nach einem der Ansprüche 1 - 5, dadurch gekennzeichnet, daß man sowohl die wässerige alkalische Lösung aus der Waschung der organischen Phase mit Alkalilauge als auch die durch Verteilung der Diacetonsorbose zwischen Wasser oder schwach alkalischen wässerigen Lösungen und einem organischen Lösungsmittel abgetrennten Nebenprodukte in den Acetonierungsprozeß zurückführt.

6. Verfahren nach einem der Ansprüche 1 - 6, dadurch gekennzeichnet, daß die aufgereinigte Diacetonsorbose für die Herstellung von Ascorbinsäure verwendet wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y,D | DE-A-2 003 067 (HOFFMANN-LA ROCHE) <br> * Seiten 10,11 * <br><br> --- | 1 | C 07 H 9/04 <br> C 07 D 307/62 |
| Y | CHEMICAL ABSTRACTS, Band 91, Nr. 8, 20. August 1979, Seite 410, Nr. 63401f, Columbus, Ohio, USA B.S. KRYUCHKOV: "Study of the separation of a mixture of diacetone sorbose and monoacetone sorbose by liquid extraction" & KHIMIYA I KHIM. TEKHNOL., PERM 1978, 143-147 * Zusammenfassung * <br><br> ----- | 1 | |

| RECHERCHIERTE SACHGEBIETE (Int. Cl. ³) |
|---|
| C 07 H 9/00 |

Der vorliegende Recherchenbericht wurde für alle Patentansprüche erstellt.

| Recherchenort | Abschlußdatum der Recherche | Prüfer |
|---|---|---|
| DEN HAAG | 29-03-1984 | VERHULST W. |